(19) Europäisches Patentamt
European Patent Office
Office européen des brevets

(11) Numéro de publication: **0 200 583 B1**

# (12) FASCICULE DE BREVET EUROPEEN

(45) Date de publication de fascicule du brevet: **12.06.91**

(51) Int. Cl.[5]: **C07D 401/04**, C07D 211/26

(21) Numéro de dépôt: **86400527.7**

(22) Date de dépôt: **12.03.86**

Le dossier contient des informations techniques présentées postérieurement au dépôt de la demande et ne figurant pas dans le présent fascicule.

## (54) Dérivés du 7-hydroxy indole, leurs sels, procédé de préparation, application à titre de médicaments, compositions les renfermant et intermédiaires.

(30) Priorité: **12.03.85 FR 8503585**

(43) Date de publication de la demande: **10.12.86 Bulletin 86/45**

(45) Mention de la délivrance du brevet: **12.06.91 Bulletin 91/24**

(84) Etats contractants désignés: **AT BE CH DE GB IT LI LU NL SE**

(56) Documents cités:
**EP-A- 0 021 924**
**EP-A- 0 071 521**

**JOURNAL OF MEDICINAL CHEMISTRY, vol. 24, no. 12, décembre 1981, pages 1475-1482, American Chemical Society; U. HACKSELL et al.: "3-Phenylpiperidines. Central dopamine-autoreceptor stimulating activity"**

(73) Titulaire: **ROUSSEL-UCLAF**
**35, boulevard des Invalides**
**F-75007 Paris(FR)**

(72) Inventeur: **Nedelec, Lucien**
**45, boulevard de l'Ouest**
**F-93340 Le Raincy(FR)**
Inventeur: **Fauveau, Patrick**
**40, avenue Camille Desmoulins**
**F-93190 Livry-Gargan(FR)**
Inventeur: **Hamon, Gilles**
**40, rue de Bagneux**
**F-92120 Montrouge(FR)**
Inventeur: **Oberlander, Claude**
**2, rue Paul Albert**
**F-75018 Paris(FR)**

(74) Mandataire: **Vieillefosse, Jean-Claude et al**
**Département des Brevets ROUSSEL UCLAF**
**B.P no 9**
**F-93230 Romainville(FR)**

**EP 0 200 583 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés du 7-hydroxy indole ainsi que leurs sels, Le procédé de préparation, l'application à titre de médicaments de ces nouveaux dérivés, Les compositions Les renfermant, et des intermédiaires.

Le brevet français n¤ 2 458 549 a déjà décrit des dérivés du 4-(pipéridin-3-yl) 1H-indole doués de propriétés stimulantes dopaminergiques marquées au niveau central.

La demande de brevet européen n¤ 71.521 décrit des dérivés du 2-oxo pyrid-3-yl ou piperidin-3-yl présentant une activité dopaminergique. Cependant ces dérivés ont une activité stimulante dopaminergique centrale alors que les produits de la présente demande sont dépourvus d'une telle activité centrale.

La présente invention a pour objet de nouveaux dérivés du 7-hydroxy indole, ainsi que leurs sels d'addition avec les acides minéraux ou organiques, caractérisés en ce qu'il répondent à la formule générale I :

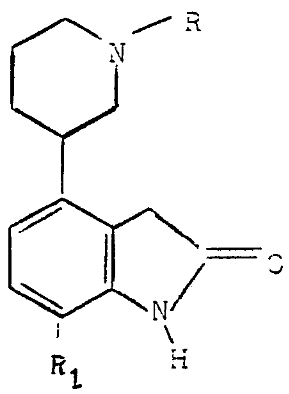

(I)

dans laquelle R représente un atome d'hydrogène, un radical alkyle renfermant de 1 à 5 atomes de carbone, cycloalkylalkyle renfermant de 4 à 7 atomes de carbone, aralkyle renfermant de 7 à 12 atomes de carbone, éventuellement substitué par un ou plusieurs radicaux choisis dans le groupe constitué par les radicaux alkyle renfermant de 1 à 5 atomes de carbone, alkoxy renfermant de 1 à 5 atomes de carbone, alkényle ou alkynyle renfermant de 3 à 5 atomes de carbone et les atomes d'halogène, étant entendu que la liaison multiple ne peut pas se trouver entre les carbones en $\alpha$ et $\beta$ de l'atome d'azote et $R_1$ représente un radical hydroxy ou alkoxy renfermant de 1 à 5 atomes de carbone, phénylalkyloxy renfermant de 7 à 9 atomes de carbone ou phénoxy.

Dans la formule générale I et dans ce qui suit, le terme radical alkyle renfermant de 1 à 5 atomes de carbone désigne, de préférence, un radical méthyl, éthyl, propyl ou isopropyl ; le terme radical cycloalkyl alkyle renfermant de 4 à 7 atomes de carbone désigne, de préférence, un radical cyclopropylméthyle ou cyclobutylméthyle ; le terme radical aralkyle renfermant de 7 à 12 atomes de carbone désigne, de préférence, un radical benzyle, phénéthyle ou naphtyl méthyle ; l'aryle peut être plurisubstitué ou, de préférence, monosubstitué ; le terme radical alkoxy renfermant de 1 à 5 atomes de carbone désigne, de préférence, un radical méthoxy, éthoxy, propoxy ou isopropoxy ; le terme atome d'halogène désigne, de préférence, un atome de chlore ou de brome ; les termes radical alkényle et alkynyle renfermant de 3 à 5 atomes de carbone désignent, de préférence, un radical allyle ou propargyle ; le terme radical phénylalkyloxy renfermant de 7 à 9 atomes de carbone désigne, de préférence, un radical benzyloxy ou phénéthyloxy.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane et éthane sulfoniques, arylsulfoniques, tels que les acides benzène et paratoluène sulfoniques et arylcarboxyliques.

Parmi les produits objet de l'invention, on peut citer notamment les dérivés répondant à la formule I ci-dessus, ainsi que leurs sels d'addition avec les acides minéraux ou organiques, caractérisés en ce que, dans ladite formule I, $R_1$ représente un radical hydroxy.

Parmi ceux-ci, on peut citer tout particulièrement les dérivés caractérisés en ce que, dans ladite formule (I), R représente un atome d'hydrogène ou un radical alkyle renfermant de 1 à 5 atomes de carbone et tout

particulièrement la 1,3-dihydro 7-hydroxy 4-(3-pipéridinyl) 2H-indol-2-one ainsi que ses sels d'addition avec les acides minéraux ou organiques.

L'invention a également pour objet un procédé de préparation des dérivés, tels que définis par la formule I ci-dessus, ainsi que de leurs sels, caractérisé en ce que l'on active sous forme d'halogénamine un dérivé de formule II :

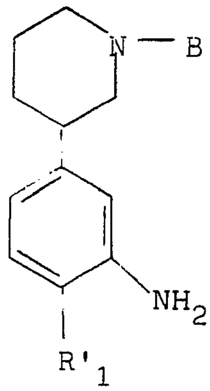

(II)

dans laquelle B représente un agent de blocage réversible d'une amine secondaire et $R'_1$ a la valeur de $R_1$ à l'exception de l'hydroxy et fait réagir l'halogénamine avec le methylthioacétate d'éthyle en présence d'un base, puis avec un acide pour obtenir un produit de formule III :

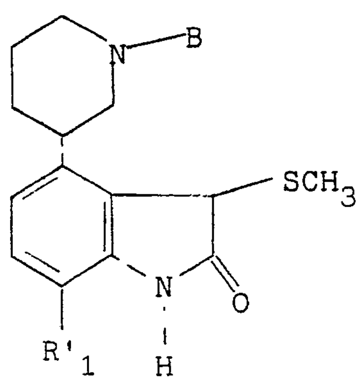

(III)

dans laquelle B et $R'_1$ ont la signification déjà indiquée, que l'on désulfure, pour obtenir un produit de formule IV:

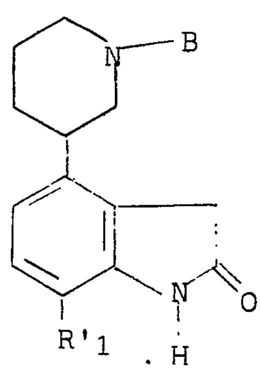

(IV)

dans laquelle B et $R'_1$ ont la signification déjà indiquée, puis fait réagir avec un agent de déblocage de l'amine secondaire, pour obtenir un produit de formule $I_A$ :

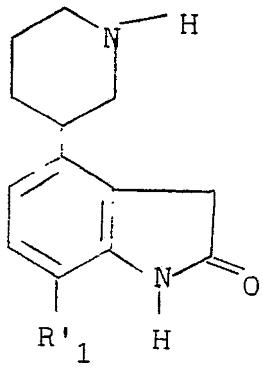

$(I_A)$

dans laquelle $R'_1$ a la signification déjà indiquée, que soit l'on isole et salifie si désiré, soit l'on soumet à l'action d'un agent de déblocage de l'hydroxyle pour obtenir un produit de formule $I_B$ :

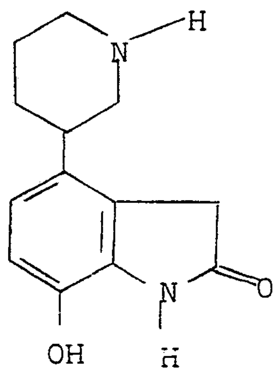

$(I_B)$

que ou bien l'on isole et salifie si désiré, ou bien l'on fait réagir avec un agent capable de greffer un radical R', R' ayant la significiation de R à l'exception de l'hydrogène, pour obtenir un produit de formule $I_C$ :

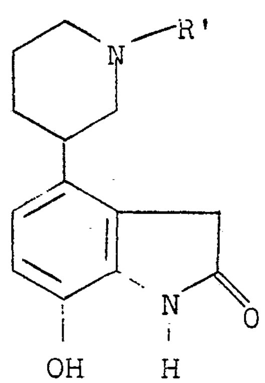

$(I_C)$

dans laquelle R' a la signification déjà indiquée, que l'on isole et, si désiré, salifie, soit l'on fait réagir ledit produit de formule $I_A$ avec un agent capable de greffer un radical R' tel que défini ci-dessus, pour obtenir un produit de formule $I_D$ :

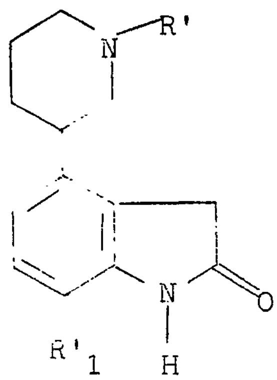

$(I_D)$

dans laquelle R' et $R'_1$ ont la signification déjà indiquée, que ou bien l'on isole et, si désiré, salifie, ou bien l'on soumet à l'action d'un agent de déblocage de l'hydroxyle pour obtenir un produit de formule $I_C$ tel que défini ci-dessus, que l'on isole et, si désiré, salifie.

L'agent B de blocage est, par exemple, un radical benzyle, et de préférence trifluoroacétyle, l'activation de l'amine aromatique est réalisée de préférence sous forme de chloramine, par exemple, à l'aide d'un hypochlorite, tel que l'hypochlorite de sodium ou de terbutyle.

La réaction de l'halogénamine avec le méthylthioacétate d'éthyle a lieu en présence d'une base telle que la triéthylamine, ou d'un alcoolate, tel que l'éthylate de sodium.

L'acide permettant la cyclisation peut être, par exemple, un acide minéral comme l'acide chlorhydrique ou l'acide bromhydrique, ou organique.

La désulfuration du produit de formule III est de préférence réalisée à l'aide de Nickel de Raney.

La méthode de déblocage de l'amine secondaire est une de celles habituellement utilisées pour de tels déblocages : l'hydrogénation catalytique dans le cas d'un groupement benzyle, ou l'hydrolyse basique, par exemple à l'aide d'une base minérale telle que la potasse ou la soude ou un carbonate tel que de sodium ou potassium ou d'un acide tel que l'acide bromhydrique ou chlorhydrique au reflux, dans le cas d'un groupement trifluoroacétyle.

Le déblocage de l'hydroxyle peut être effectué à chaud, par exemple, à l'aide d'acide bromhydrique ou de chlorhydrate de pyridine au reflux; on peut ainsi obtenir directement le sel. Il peut être effectué à froid, par exemple, à l'aide de tribromure de bore ; on obtient ainsi le produit $I_B$ ou $I_C$ sous forme de base. Il peut être aussi effectué par hydrogénation catalytique dans le cas où l'hydroxyle est bloqué sous forme d'éther benzylique.

L'agent capable de greffer un radical R' est de préférence un halogénure de formule XI :

Hal-R" (XI)

dans laquelle R" a la signification de R déjà indiquée, à l'exception de l'hydrogène et du méthyle.

L'halogénure de formule (XI) peut être un chlorure ou un bromure, mais de préférence un iodure. Il réagit avantageusement avec les amines secondaires de formules $(I_A)$ et $(I_B)$ en présence d'un agent fixateur d'acide, comme, par exemple, un carbonate alcalin tel que le carbonate de potassium dans un solvant tel que le diméthylformamide.

Pour fixer un radical R' sur les dérivés de formules $(I_A)$ et $(I_B)$, on peut opérer également par action desdits dérivés de formules $(I_A)$ et $(I_B)$ avec un dérivé (selon le cas un aldéhyde ou une cétone) de formule (XII) :

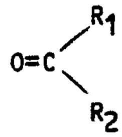

(XII)

dans laquelle $R_1$ et $R_2$ sont tels que le radical

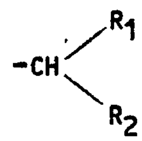

a la signification de R déjà indique à l'exception de l'hydrogène, en présence d'un agent de réduction (borohydrure ou cyanoborohydrure alcalin, tel que borohydrure ou cyanoborohydrure de sodium ou hydrogénation catalytique). C'est ainsi, par exemple, que si le dérivé de formule (XII) est le formaldéhyde ou l'acétaldéhyde, on obtient respectivement pour R', les valeurs méthyle et éthyle.

L'invention a aussi pour objet une variante du procédé ci-dessus décrit, de préparation des dérivés tels que définis par la formule (I) ci-dessus ainsi que le leurs sels, caractérisée en ce que l'on active sous forme d'halogénamine, un dérivé de formule (II') :

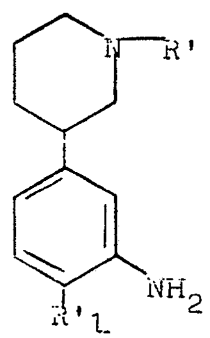

(II')

dans laque R' et $R'_1$ ont la signification de R et $R_1$ respectivement, à l'exception de l'hydrogène pour R et de hydroxy pour $R_1$ et fait réagir l'halogénamine avec le méthylthioacétate d'éthyle en présence d'une base, puis avec un acide, pour obtenir un produit de formule (III') :

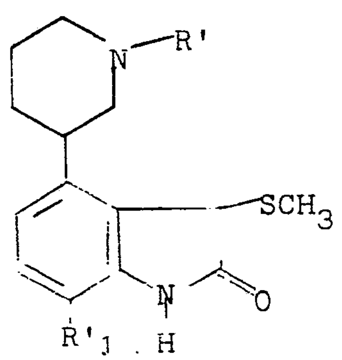

(III')

dans laquelle R' et $R'_1$ ont la signification déjà indiquée, que l'on désulfure, pour obtenir un produit de formule ($I_D$) tel que défini ci-dessus, que soit l'on isole et, si désiré, salifie, soit l'on soumet à l'action d'un agent de déblocage de l'hydroxyle, pour obtenir un produit de formule ($I_C$) tel que défini ci-dessus, que ou bien l'on isole et, si désiré, salifie, ou bien dans le cas où R' représente un radical benzyle, l'on soumet à l'action d'un agent de déblocage du benzyle, pour obtenir un produit de formule ($I_B$) tel que défini ci-dessus, soit dans le cas où R' représente un radical benzyle, l'on soumet ledit produit de formule ($I_D$), à l'action d'un agent de déblocage du benzyle, pour obtenir un produit de formule ($I_A$) tel que défini ci-dessus, que ou bien l'on isole et, si désiré, salifie, ou bien l'on soumet à l'action d'un agent de déblocage de l'hydroxyle, pour obtenir un produit de formule ($I_B$) tel que défini ci-dessus, que l'on isole et, si désiré, salifie.

Les conditions opératoires pour cette variante sont analoques à celles du premier procédé décrit.

Les produits de départ de formule (II) peuvent être préparés par réaction du magnésien d'un produit de formule (V) :

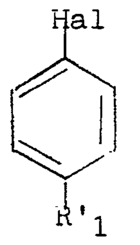

(V)

dans laquelle $R'_1$ a la signification déjà indiquée et Hal représente un atome de chlore ou de brome, avec un dérivé de la pipéridone de formule (VI):

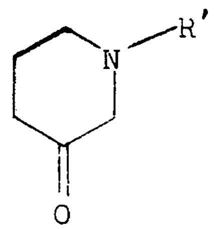

(VI)

dans laquelle R' a la signification déjà indiquée, pour obtenir un produit de formule (VII) :

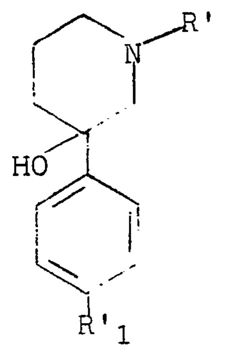

(VII)

dans laquelle R' et $R'_1$ ont la signification déjà indiquée, que l'on déshydrate, pour obtenir un produit de formule (VIII) :

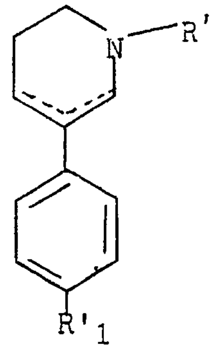

(VIII)

dans laquelle R' et $R'_1$ ont la signification déjà indiquée et le pointillé signifie la présence d'un mélange d'isomères 1,2,5,6 et 1,4,5,6-tétrahydropyridines, que l'on soumet à une hydrogénation catalytique pour obtenir un produit de formule (IX) :

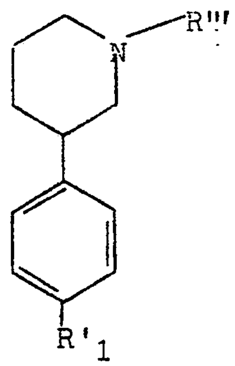

(IX)

dans laquelle R'$_1$ a la signification déjà indiquée et R''' a la signification de R à l'exception du radical benzyle, que soit dans le cas où R''' ne représente pas un atome d'hydrogène, l'on soumet à une nitration, pour obtenir un produit de formule (X) :

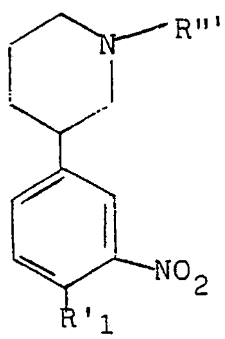

(X)

dans laquelle R''' et R'$_1$ ont la signification déjà indiquée, que l'on réduit, pour obtenir un produit de formule (II') tel que défini ci-dessus, mais dans laquelle R' ne peut pas représenter un radical benzyle, soit dans le cas où R''' représente un atome d'hydrogène, l'on fait réagir avec un agent susceptible de bloquer l'amine de manière réversible, pour obtenir un produit de formule (IX') :

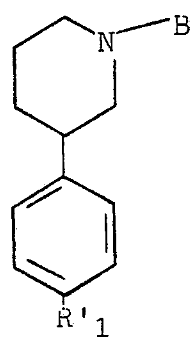

(IX')

dans laquelle B et R'$_1$ ont la significaton déjà indiquée que l'on traite comme pour le produit de formule (IX) ci-dessus (nitration puis réduction) de manière à obtenir un produit de formule (II) tel que défini ci-dessus.

La formation du magnésien du produit de formule (V) est réalisée de préférence en faisant réagir du magnésium sur le produit de formule (V), de préférence dans le tétrahydrofuranne, en présence d'une faible quantité de dibromoéthane (comme catalyseur).

La réaction du magnésien avec le dérivé de pipéridone de formule (VI) est faite de préférence à basse température, aux environs de 0¤C dans le tétrahydrofuranne.

La déshydratation du produit de formule (VII) est réalisée de préférence au reflux du benzène, en présence d'acide paratoluène sulfonique. On peut utiliser également le chlorure de thionyle ou l'anhydride phosphorique ou un acide minéral tel que l'acide chlorhydrique au reflux.

L'hydrogénation catalytique du produit de formule (VIII) est réalisée de préférence en présence de

palladium.

La nitration du produit de formule (IX) ou (IX') est réalisée de préférence à l'aide d'un mélange acide trifluoroacétique - acide nitrique fumant. On peut également faire réagir l'acide nitrique fumant dans l'acide sulfurique ou l'anhydride acétique.

L'agent susceptible de bloquer de façon réversible l'amine de formule (IX) est l'anhydride trifluoroacétique, de préférence en présence d'une base, dans le cas où l'on souhaite que B représente un radical trifluoroacétyle ou un halogénure de benzyle, de préférence bromure ou chlorure de benzyle, en présence d'une base, dans le cas où l'on souhaite que B représente un radical benzyle.

La réduction du groupe nitro est effectuée de préférence par réduction catalytique. De manière générale, on peut opérer par action de chlorure stanneux ou ferreux ou d'acétylacétonate de cuivre en présence de borohydrure de sodium.

Les dérivés de formule (I) présentent un caractère basique. On peut avantageusement préparer les sels d'addition des dérivés de formule (I) en faisant réagir, en proportions sensiblement stoechiométriques, un acide minéral ou organique avec ledit dérivé de formule (I). Les sels peuvent être préparés sans isoler les bases correspondantes.

Les dérivés, objet de la présente invention, possèdent de très intéressantes propriétés pharmacologiques; ils sont doués notamment de remarquables propriétés dopaminergiques exerçant des effets cardiovasculaires périphérique (propriétés vasodilatatrices périphériques), inotropes positiveset très peu d'effets centraux. Ils ont une remarquable affinité pour les récepteurs dopaminergiques post-synaptiques très dissociée de l'affinité pour les récepteurs $\alpha$-adrénergiques.

Ces propriétés sont illustrées plus loin dans la partie expérimentale.

Ces propriétés justifient l'utilisation des dérivés du 7-hydroxy indole ainsi que de leurs sels, à titre de médicaments.

La présente demande a ainsi également pour objet l'application, à titre de médicaments, des dérivés du 7-hydroxy indole tels que définis par la formule (I) ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés du 7-hydroxy indole répondant à la formule (I) dans laquelle $R_1$ représente un radical hydroxy, ainsi que par leurs sels d'addition avec les acides pharmaceutiquement acceptables.

Parmi ceux-ci, on retient notamment ceux répondant à la formule (I) dans laquelle R représente un atome d'hydrogène ou un radical alkyle renfermant de 1 à 5 atomes de carbone, ainsi que leurs sels d'addition avec les acides pharmaceutiquement acceptables.

Parmi ces derniers, on retient tout particulièrement :

- la 1,3-dihydro 7-hydroxy 4-(3-pipéridinyl)2H-indol-2-one, ainsi que ses sels d'addition avec les acides pharmaceutiquement acceptables.

Les médicaments selon l'invention trouvent leur emploi, par exemple, dans le traitement des accidents vasculaires cérébraux, des troubles circulatoires périphériques tels que rétiniens ou néphrétiques ou des artériopathies des membres inférieurs. Ils sont également utilisables dans le traitement des insuffisances cardiaques de diverses étiologies.

La dose usuelle, variable selon le dérivé utilisé, le sujet et l'affection en cause peut être, par exemple, de 5 mg à 200 mg par jour.

Par voie orale, chez l'homme, le dérivé de l'exemple 3peut être administré à la dose quotidienne de 10 mg à 200 mg, par exemple, pour le traitement de l'insuffisance cardiaque, soit environ de 0,15 mg à 3 mg par kilogramme de poids corporel.

L'invention a également pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magésium, le beurre de cacao, les véhicules aqueux ou non, les corps gras d'origine animale ou végétale, les dérivés paraffiniques, les glycols, les divers agents

mouillants, dispersants ou émulsifiants, les conservateurs.

L'invention s'étend, en outre, aux produits industriels nouveaux de formules (II) et (II') :

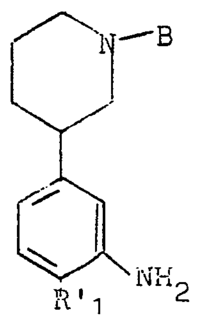

(II)

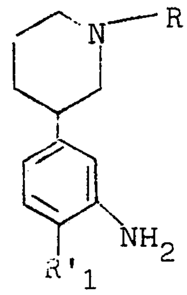

(II')

dans lesquelles B, R" et $R'_1$ ont la signification déjà indiquée ci-dessus et leurs halogénamines.

**Exemple 1 : 1,3-dihydro 7-méthoxy 4-(3-pipéridinyl)2H-indol-2-one.**

Stade A : 1,3-dihydro 7-méthoxy 3-méthylthio 4-/1-(trifluoroacétyl)3-pipéridinyl/2H-indol-2-one.

On place sous agitation et atmosphère inerte 604 mg de 3-(3-amino 4-méthoxy phényl)1-trifluoroacétyl pipéridine dans 20 cm3 de chlorure de méthylène et 0,26 cm3 de méthylthioacétate d'éthyle, refroidit à -65¤C, ajoute en 5 minutes, 0,24 cm3 d'hypochlorite de tertbutyle, laisse sous agitation pendant une heure, ajoute goutte à goutte 0,29 cm3 de triéthylamine, laisse revenir à température ambiante et ajoute 5 cm3 d'acide chlorhydrique en solution aqueuse 2N. On laisse une heure sous agitation, décante, lave à l'eau, sèche, filtre, amène à sec sous pression réduite, purifie par chromatographie sur silice (éluant benzène - acétate d'éthyle 7-3) et obtient 580 mg de produit attendu que l'on recristallise dans l'éthanol. F≈189¤C.

Stade B : 1,3-dihydro 7-méthoxy 4-/1-(trifluoroacétyl)3-pipéridinyl/2H-indol-2-one.

On ajoute sous agitation et atmosphère inerte, 70 g de nickel de Raney à une solution de 7,7 g du produit obtenu au stade A dans 200 cm3 de tétrahydrofuranne en maintenant la température à environ 20¤C et laisse une heure sous agitation.

On filtre, rince au tétrahydrofuranne, amène à sec sous pression réduite, reprend le résidu au chlorure de méthylène, lave à l'eau, sèche, amène à sec sous pression réduite et obtient 6,6 g du produit attendu que l'on recristallise dans l'éthanol. F = 174¤C.

Stade C : 1,3-dihydro 7-méthoxy 4-(3-pipéridinyl)2H-indol-2-one.

On agite pendant une heure sous atmosphère inerte, 6 g du produit obtenu au stade précédent dans 150 cm3 de méthanol et 60 cm3 de soude 2N, extrait au chlorure de méthylène, lave à l'eau, sèche, filtre, amène à sec sous pression réduite et obtient 4,3 g du produit attendu que l'on recristallise dans l'éthanol. F 100¤C, puis 168¤C.

Analyse pour $C_{14}H_{18}N_2O_2$ = 246,30

| | | | |
|---|---|---|---|
| Calculé | C% 68,26 | H%7,36 | N%11,38 |
| Trouvé | 68,2 | 7,4 | 11,1. |

Préparation de la 3-(3-amino 4-méthoxyphényl)1-trifluoroacétyl pipéridine de départ pour l'exemple 1.

Stade 1 : 3-(4-méthoxyphényl)1-(phénylméthyl)-3-pipéridinol.

- Préparation du magnésien :

On ajoute à 5,25 g de tournures de magnésium, 1 cristal d'iode que l'on sublime par chauffage, ajoute après refroidissement 10 cm3 de tétrahydrofuranne, 1 cm3 de p-bromoanisole et 0,3 cm3 de dibromoéthane. On amorce la réaction par chauffage puis ajoute goutte à goutte un mélange de 25 cm3 de p-bromoanisole et 120 cm3 de tétrahydrofuranne, chauffe encore une heure au reflux et laisse reposer

pendant 16 heures.

- Condensation :

On refroidit à l'aide d'un bain de glace, 100 cm3 de la solution obtenue ci-dessus, ajoute goutte à goutte sous agitation et atmosphère inerte en 1 heure vingt minutes, 22 g de N-benzyl 3-pipéridone en solution dans 100 cm3 de tétrahydrofuranne, laisse remonter à température ambiante, laisse 2 heures sous agitation, refroidit et ajoute goutte à goutte 150 cm3 de solution aqueuse saturée de chlorure d'ammonium. On extrait à l'acétate d'éthyle, lave plusieurs fois à l'aide d'une solution aqueuse d'acide chlorhydrique 2N, alcalinise à l'aide de lessive de soude, extrait au chlorure de méthylène, lave à l'eau, sèche et amène à sec sous pression réduite. L'huile obtenue est chromatographiée sur silice (éluant benzène - acétate d'éthyle 1-1) et on obtient 23 g du produit attendu. F≃58¤C.

Stade 2 : Mélange de 3-(4-méthoxy phényl) 1-(phénylméthyl)1,2,5,6-tétrahydropyridine et de 3-(4-méthoxyphényl)1-(phénylméthyl)1,4,5,6-tétrahydropyridine.

On porte pendant 2 heures au reflux sous agitation et atmosphère inerte, 7 g du produit obtenu au stade 1, 10,15 g d'acide paratoluène sulfonique et 300 cm3 de benzène, refroidit, lave à la soude 2N, sèche, amène à sec sous pression réduite, purifie par chromatographie sur silice (éluant benzène - acétate d'éthyle 1-1) et obtient 5,6 g du produit attendu,
soit 1 g d'isomère 1,4,5,6-tétrahydropyridine, F = 74¤C
et 4,6 g d'isomère 1,2,5,6-tétrahydropyridine, F ≃ 98¤C.

Stade 3 : 3-(4-méthoxyphényl)1-(trifluoroacétyl)pipéridine.
Hydrogénation :

On fait absorber 2,5 litres d'hydrogène à 15,5 g du mélange obtenu comme au Stade 2 en solution dans 250 cm3 d'acide acétique, en présence de 3,6 g de palladium à 10%, filtre, lave à l'acide acétique, amène à sec sous pression réduite, ajoute 100 cm3 d'eau, refroidit à l'aide d'un bain de glace, alcalinise à l'ammoniaque, extrait au chlorure de méthylène, sèche, amène à sec sous pression réduite et obtient 10,5 g d'huile.

Trifluoroacétylation :

On refroidit à 0¤C l'huile ci-dessus en solution dans 60 cm3 de chlorure de méthylène, ajoute goutte à goutte 15,6 cm3 d'anhydride trifluoroacétique, laisse 1 heure sous agitation à température ambiante, amène à sec sous pression réduite pour éliminer l'excès d'anhydride, reprend le résidu au chlorure de méthylène, lave à l'aide d'une solution saturée de bicarbonate de sodium, sèche, amène à sec sous pression réduite, ajoute quelques cm3 d'éther de pétrole, essore le produit cristallisé et obtient 14,3 g du produit attendu. PF¤ 88¤C.

Stade 4 : 3-(4-méthoxy 3-nitrophényl) 1-(trifluoroacétyl) pipéridine.

On ajoute goutte à goutte sous agitation 2,24 cm3 d'acide nitrique fumant à 14,3 g du produit obtenu au stade précédent dans 50 cm3 d'acide trifluoroacétique à 0¤c, laisse remonter à température ambiante et maintient sous agitation pendant 1 heure ; on verse sur de la glace, extrait au chlorure de méthylène, lave à l'eau, sèche, amène à sec sous pression réduite, purifie par chromatographie sur silice (éluant benzène acétate d'éthyle 9-1) et obtient 12,3 g du produit attendu que l'on recristallise dans l'éther isopropylique. F = 96¤C.

Stade 5 : 3-(3-amino 4-méthoxyphényl)1-(trifluoroacétyl)pipéridine.

On fait absorber 2,4 litres d'hydrogène à une solution de 12,3 g du produit obtenu au stade précédent dans 400 cm3 d'éthanol en présence de 3,7 g de palladium à 10 %, filtre, lave à l'éthanol, amène à sec sous pression réduite. On empâte le résidu solide dans l'éther isopropylique, essore, sèche et obtient 9,4 g du produit attendu. F = 88¤C

**Exemple 2 : 1,3-dihydro 7-méthoxy 4-(1-propyl 3-pipéridinyl)2H-indol-2-one.**

On agite sous atmosphère inerte pendant 1 heure 30 minutes, 2 g du produit de l'exemple 1 dans 40 cm3 de diméthylformamide avec 3,39 g de carbonate de potassium et 1,6 cm3 d'iodure de propyle. On ajoute de l'eau, extrait au chlorure de méthylène, lave à l'eau, sèche, amène à sec sous pression réduite et purifie le résidu par chromatographie sur silice (éluant : chlorure de méthylène - méthanol 9-1) ; on obtient 1,6 g du produit attendu. F≃120¤C.

Analyse pour $C_{17}H_{24}N_2O_2$ = 288,38

| | | | |
|---|---|---|---|
| Calculé C% 70,20 | H% 8,39 | N% 9,71 | |
| Trouvé 70,5 | 8,5 | 9,6. | |

**Exemple 3 : Bromhydrate du 1,3-dihydro 7-hyroxy 4-(3-pipéridinyl)2H-indol-2-one.**

On chauffe au reflux pendant 2 heures 30 minutes sous atmosphère inerte, 1,5 g du produit de l'exemple 1 dans 15 cm3 d'acide bromhydrique concentré (d = 1,49), amène à sec sous pression réduite, reprend le résidu au benzène, concentre à sec, ajoute 2 cm3 d'éthanol, essore les cristaux obtenus et les sèche sous pression réduite. On purifie par recristallisation dans l'éthanol et obtient 1,4 g de produit attendu. F¤ 190¤C, puis 250¤C.

Analyse pour $C_{13}H_{16}N_2O_2HBr$ : 313,21

| | | | |
|---|---|---|---|
| Calculé C% 49,85 | H% 5,47 | N% 8,94 | Br% 25,52 |
| Trouvé 49,7 | 5,7 | 8,8 | 25,3. |

**Exemple 4 : Bromhydrate de 1,3-dihydro 7-hydroxy 4-(1-propyl 3-pipéridinyl)2H-indol-2-one.**

On porte au reflux pendant 2 heures sous agitation et atmosphère inerte, 1,55 g du produit de l'exemple 2 dans 15 cm3 d'acide bromhydrique à 48%, amène à sec sous pression réduite, fait cristalliser l'huile obtenue dans l'éthanol, essore, lave à l'éther, sèche, purifie par recristallisation dans le méthanol et obtient 1,31 g du produit attendu. F = 210¤C.

Analyse pour $C_{16}H_{22}N_2O_2HBr$ : 355,29

| | | | |
|---|---|---|---|
| Calculé C% 54,08 | H% 6,53 | N% 7,89 | Br% 22,49 |
| Trouvé 53,9 | 6,7 | 7,7 | 22,3. |

**Exemple 5 : 1,3-dihydro 7-méthoxy 4-(1-méthyl 3-pipéridinyl) 2H-indol-2-one et chlorhydrate.**

On dissout sous agitation et atmosphère inerte 4,5 g de produit de l'exemple 1 dans 100 cm3 de méthanol, refroidit à environ 5¤C, ajoute goutte à goutte 3,4 cm3 d'aldéhyde formique à 40 %, agite 1 heure, ajoute par petites fractions 1,8 g de borohydrure de sodium à 95 % et maintient sous agitation pendant 1 heure. On laisse revenir à température ambiante, dilue avec de l'eau glacée, extrait au chlorure de méthylène, sèche et amène à sec sous pression réduite. On essore, lave à l'éther, sèche et obtient 4 g de produit attendu sous forme de base. F≃170¤C.

Formation du chlorhydrate.

On dissout en tiédissant 1,6 g de base dans 30 cm3 de méthanol, ajoute 5 cm3 de méthanol chlorhydrique, évapore 15 cm3 de méthanol et laisse cristalliser lentement en refroidissant à l'aide d'un bain de glace ; on essore les cristaux obtenus, les lave à l'éther, les sèche à 80¤C sous pression réduite et obtient 1,44 g du produit attendu. F>250¤C.

Analyse pour $C_{15}H_{20}N_2O_2,HCl$ = 296,80

| | | | | |
|---|---|---|---|---|
| Calculé | C% 60,70 | H% 7,13 | N% 9,44 | Cl% 11,94 |
| Trouvé | 60,7 | 7,2 | 9,3 | 11,8. |

**Exemple 6 : Bromhydrate de 1,3-dihydro 7-hydroxy 4-(1-méthyl 3-pipéridinyl) 2H-indol-2-one.**

On porte au reflux pendant 3 heures sous agitation et atmosphère inerte, 2,2 g de produit de l'exemple 5 dans 25 cm3 d'acide bromhydrique à 48 %, amène à sec sous pression réduite, reprend le résidu à l'éthanol, essore le produit cristallisé obtenu, le lave à l'acétone, le sèche sous pression réduite et obtient 2,5 g de produit attendu que l'on recristallise dans le méthanol. F >250¤C.
Analyse pour $C_{14}H_{18}N_2O_2$, HBr = 327,23

| | | | | |
|---|---|---|---|---|
| Calculé | C% 51,38 | H% 5,85 | N% 8,58 | Br% 24,42 |
| Trouvé | 51,2 | 5,8 | 8,6 | 24,2. |

**Exemple 7 :**

On a préparé des comprimés répondant à la formule :
- bromhydrate de 1,3-dihydro 7-hydroxy 4-(3-pipéridinyl)2H-indol-2-one..........................................................5mg
- Excipient q.s.pour un comprimé terminé à....................100mg.
  (Détail de l'excipient : lactose, amidon, talc, stéarate de magnésium).

**Exemple 8 :**

On a préparé des comprimés répondant à la formule :
- bromhydrate de 1,3-dihydro 7-hydroxy 4-(1-propyl 3-pipéridinyl)2H-indol-2-one....................................................10mg
- Excipient q.s. pour un comprimé terminé à...............100mg.
  (Détail de l'excipient : lactose, amidon, talc, stéarate de magnésium).

**ETUDE PHARMACOLOGIQUE**

**1)Comportement de rotation après lésion unilatérale du faisceau nigrostriatal par la 6-hydroxydopamine.**

Technique :

Des rats mâles de 220 g environ sont lésés dans le faisceau dopaminergique nigrostrié selon la méthode du Ungerstedt (Acta physiol.Scand. 1971, 82 suppl. 367-69 93) modifiée, par injection unilatérale de 9,2 $\mu$g de chlorhydrate de 6-hydroxydopamine en solution dans 4 $\mu$l de soluté physiologique contenant 0,5 mg/ml d'acide ascorbique.

Les produits étudiés sont administrés par voie intrapéritonéale. Les animaux traités au nombre de 6 en moyenne, sont placés individuellement dans un rotomètre qui permet de compter le nombre de rotations effectuées par chaque animal dans le sens opposé au côté lésé. Chaque essai est poursuivi pendant un temps au moins égal à une heure trente minutes.

Dans les conditions de l'expérience les résultats suivants ont été obtenus :

A la dose de 5 mg/kg, le produit de l'exemple 3 n'induit pratiquement par de rotations. Il est donc substantiellement dépourvu d'activité stimulante dopaminergique au niveau central.

**2) Comportements stéréotypés.**

Les essais sont réalisés sur des lots de 5 rats mâles de 150 à 180 g. Chaque animal est placé individuellement dans une cage grillagée (29 x 25 x 17 cm) contenant quelques débris de frisure de bois.

Le produit étudié est administré par voie intrapéritonéale.

Le comportement des animaux est noté de demi-heure en demi-heure pendant 5 heures avec la cotation préconisée par HALLIWELL et Coll. (Brit.J.Pharmacol.1964, 23, 330-350).

L'animal est endormi (0), il est éveillé, mais immobile (1), il tourne dans la cage (2), il en renifle le couvercle (3), il en lèche les parois (4) il touche les copeaux ou les barreaux de la cage avec les dents (5), il mord les copeaux ou les barreaux de la cage (6).

Le somme des scores par lot relevée à différents délais après l'administration du produit étudié est déterminée.

A la dose de 50 mg/kg, le produit de l'exemple 3 n'induit pratiquement pas de mouvements stéréotypés. Il est donc substantiellement dépourvu d'action agoniste dopaminergique au niveau central.

**3) Détermination de l'activité hypotensive.**

L'activité hypotensive a été étudiée sur des rats mâles de souche Wistar pesant 300 g environ et anesthésiés au pentobarbital sodique (50 mg/kg par voie intrapéritonéale).

Le produit testé a été administré par voie intraveineuse dans la veine jugulaire.

La pression artérielle carotidienne a été mesurée avant et après administration du produit testé.

Le produit de l'exemple n¤3 a un effet négligeable sur la pression artérielle à la dose de 1 mg/kg.

**4) Test de mise en évidence d'une activité vasodilatatrice directe (ou activité post-synaptique relaxante vasculaire).**

Les rats de race Sprague-Dawley d'un poids de 320-350 g sont anesthésiés au pentobarbital (50 mg/kg par injection intrapéritonéale). Après mise en place d'un cathéter carotidien destiné à la mesure de la pression artérielle et d'un cathéter jugulaire destiné à l'injection des molécules à étudier ainsi que des antagonistes éventuels, les animaux sont démédullés à l'aide d'une tige d'acier. Cette tige est introduite par le trou orbitaire droit et descendue sur toute la longueur de la moelle épinière. Les animaux sont ensuite placés immédiatement sous respiration assistée. Quelques minutes après stabilisation de la pression artérielle, les animaux reçoivent une perfusion intraveineuse d'angiotensine II de 50$\mu$g/kg/min de façon à augmenter leur pression moyenne d'environ 100mm de mercure. Après obtention d'un plateau, la substance à étudier est injectée par voie intraveineuse chaque 2 minutes et en doses cumulées. Les pourcentages de baisse de la pression artérielle moyenne ainsi obtenus permettent de tracer une courbe dose-réponse reflétant l'activité vasodilatatrice de la molécule étudiée. Le traitement préalable des animaux par différents antagonistes permet de cerner le mécanisme d'action de cette molécule.

Les produits sont testés entre 0,001 et 1 mg/kg. On utilise comme antagonistes la dompéridone et le propanolol.

On peut conclure que le produit de l'exemple n¤3 a un effet vasodilatateur dose dépendant dès 0,001 mg/kg et que cet effet est d'origine dopaminergique et - adrénergique.

**5) Test de mise en evidence de l'activité inotrope positive d'une molécule chez le rat anesthésié.**

Les rats de race Sprague-Dawley d'un poids de 300 à 350 g sont anesthésiés au pentobarbital (50 mg/kg par injection intrapéritonéale). Un cathéter est mis en place dans le ventricule gauche afin de mesurer le pression intraventriculaire cardiaque et la dP/dt VG (ou vitesse de variation de la pression intraventriculaire). Un second cathéter artériel, placé dans l'artère fémorale permet d'enregistrer la pression artérielle systémique et la fréquence cardiaque. Les produits à étudier ainsi que les antagonistes éventuels sont injectés par un cathéter placé dans une veine jugulaire. Ce modèle permet ainsi d'évaluer in vivo l'effet de diverses substances sur la force de contraction cardiaque en fonction de la dose et en fonction du temps. Les substances qui possèdent un effet inotrope positif, augmentent la dP/dt, la pression ventriculaire maximale ainsi que le pression différentielle.

Le produit de l'exemple 3 administré à la dose de 10 à 100$\mu$g/kg provoque une augmentation de la dP/dt et de la pression ventriculaire maximale qui n'est par ailleurs par bloquée par le propanolol. Le produit de l'exemple 3 possède donc des propriétés inotropes positives.

**6) Test de mise en évidence d'une activité présynaptique au niveau vasculaire.**

Les rats de race Sprague-Dawley d'un poids de 320-350 g sont anesthésiés au pentobarbital (50 mg/kg par injection intrapéritonéale). Après mise en place d'un cathéter carotidien destiné à la mesure de la pression artérielle et d'un cathéter jugulaire destiné à l'injection des molécules à étudier, les animaux sont démédullés à l'aide d'une tige d'acier inoxydable introduite par le trou orbitaire droit. Cette tige est isolée sur toute sa longueur à l'exception des cinq derniers centimètres. Aussitôt après destruction de la moelle,

les animaux sont placés sous respiration assistée. Après une période de repos d'environ 20 minutes, les animaux sont soumis à des stimulations électriques (appliquées entre le tige de démédullation et une électrode indifférente placée sous la peau de la région de la cuisse) (impulsions de 2msec., fréquence 10 Hz, amplitude environ 20V) de 30 sec., chaque 5 minutes. Après stabilisation des réponses, les substances à étudier sont administrées par voie intraveineuse. L'altération de l'amplitude des réponses pressives à la stimulation électrique peut résulter d'une action présynaptique (une diminution de réponse traduisant une diminution de libération de noradrénaline) ou d'une action vasculaire directe ou post-synaptique (effet α-bloqueur, vasodilatateur direct...). L'adjonction de bloqueurs appropriés et la combinaison de cette technique avec la modification des courbes dose-réponse à des agents vasoconstricteurs comme la noradrénaline permet de préciser le profil pharmacologique de la molécule étudiée.

Le produit de l'exemple 3 provoque une diminution des réponses qui n'est pas bloquée par le sulpiride. Cette diminution des réponses paraît donc liée à une activité essentiellement post-synaptique.

**7) Etude de la toxicité aiguë.**

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants :

| Produits de l'exemple | $DL_0$ en mg/kg |
|---|---|
| | 0 |
| | 0 |
| 3 | 400 |
| 4 | 400 |

**Revendications**

1. Les dérivés du 7-hydroxy indole ainsi que leurs sels d'addition avec les acides minéraux ou organiques, caractérisés en ce qu'ils répondent à la formule générale (I) :

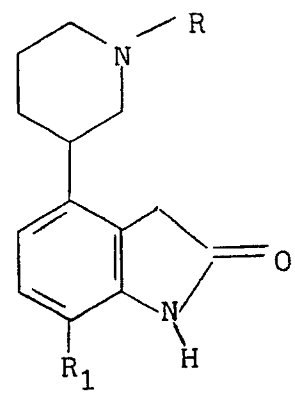

(I)

dans laquelle R représente une atome d'hydrogène, un radical alkyle renfermant de 1 à 5 atomes de

carbone, cycloalkylalkyle renfermant de 4 à 7 atomes de carbone, aralkyle renfermant de 7 à 12 atomes de carbone, éventuellement substitué par un ou plusieurs radicaux choisis dans le groupe constitué par les radicaux alkyle renfermant de 1 à 5 atomes de carbone, alkoxy renfermant de 1 à 5 atomes de carbone, alkényle ou alkynyle renfermant de 3 à 5 atomes de carbone et les atomes d'halogène, étant entendu que la liaison multiple ne peut pas se trouver entre les carbones en $\alpha$ et $\beta$ de l'atome d'azote et $R_1$ représente un radical hydroxy, alkoxy renfermant de 1 à 5 atomes de carbone, phénylalkyloxy renfermant de 7 à 9 atomes de carbone ou phénoxy.

**2.** Les dérivés du 7-hydroxy indole tels que définis par la formule (I) de la revendication 1 ainsi que leurs sels d'addition avec les acides minéraux ou organiques, caractérisés en ce que $R_1$ représente un radical hydroxy.

**3.** Les dérivés du 7-hydroxy indole selon le revendication 2 ainsi que leurs sels d'addition avec les acides minéraux ou organiques, caractérisés en ce que R représente un atome d'hydrogène ou un radical alkyle renfermant de 1 à 5 atomes de carbone.

**4.** Le dérivé de formule (I) selon la revendication 1 dont le nom suit :
- la 1,3-dihydro 7-hydroxy 4-(3-pipéridinyl)2H-indol-2-one ainsi que ses sels d'addition avec les acides minéraux ou organiques.

**5.** Procédé de préparation des dérivés du 7-hydroxy indole tels que définis par la formule (I) de la revendication 1 ainsi que de leurs sels, caractérisé en ce que l'on active sous forme d'halogénamine un dérivé de formule (II) :

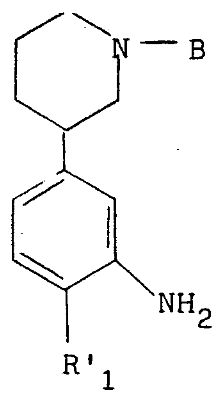

(II)

dans laquelle B représente un agent de blocage réversible d'une amine secondaire et $R'_1$ a la valeur de $R_1$ à l'exception de l'hydroxy et fait réagir l'halogénamine avec le méthylthioacétate d'éthyle en présence d'une base, puis avec un acide pour obtenir un produit de formule III :

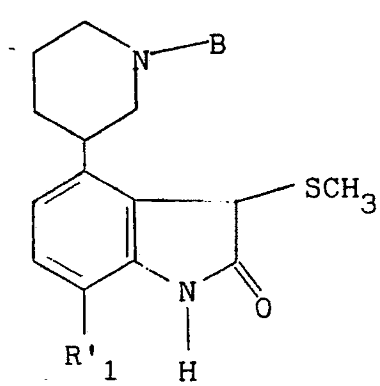

(III)

dans laquelle B et $R'_1$ ont la signification déjà indiquée, que l'on désulfure, pour obtenir un produit de formule IV :

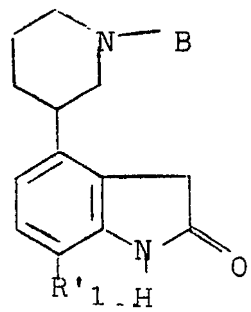

(IV)

dans laquelle B et R'$_1$ ont la signification déjà indiquée, puis fait réagir avec un agent de déblocage de l'amine secondaire, pour obtenir un produit de formule $I_A$ :

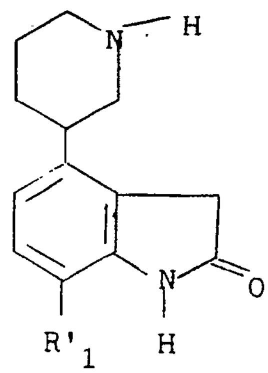

($I_A$)

dans laquelle R'$_1$ a la signification déjà indiquée, que soit l'on isole et salifie si désiré, soit l'on soumet à l'action d'un agent de déblocage de l'hydroxyle, pour obtenir un produit de formule $I_B$ :

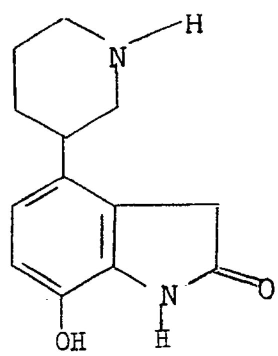

($I_B$)

que ou bien l'on isole et salifie si désiré, ou bien l'on fait réagir avec un agent capable de greffer un radical R', R' ayant la signification de R à l'exception de l'hydrogène, pour obtenir un produit de formule $I_C$ :

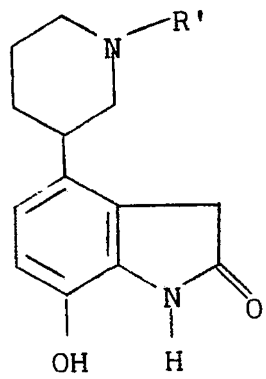

($I_C$)

dans laquelle R' a la signification déjà indiquée, que l'on isole et, si désiré, salifie, soit l'on fait réagir ledit produit de formule $I_A$ avec un agent capable de greffer un radical R' tel que défini ci-dessus, pour obtenir un produit de formule $I_D$ :

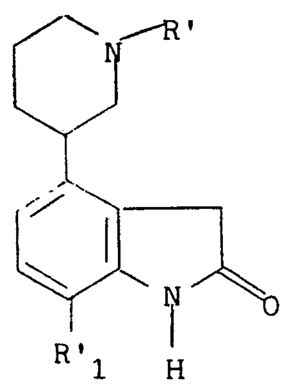

($I_D$)

dans laquelle R' et $R'_1$ ont la signification déjà indiquée, que ou bien l'on isole et si désiré, salifie, ou bien l'on soumet à l'action d'un agent de déblocage de l'hydroxyle pour obtenir un produit de formule $I_C$ tel que défini ce-dessus, que l'on isole et, si désiré, salifie.

6. Procédé selon la revendication 5, caractérisé en ce que :
   - l'agent de blocage de l'amine secondaire est un radical trifluoroacétyle ;
   - l'halogénamine est une chloramine.

7. Variante du procédé de préparation des dérivés du 7-hydroxy indole tels que définis par la formule I de la revendication 1 ainsi que de leurs sels, caractérisée en ce que l'on active sous forme d'halogénamine un dérivé de formule II' :

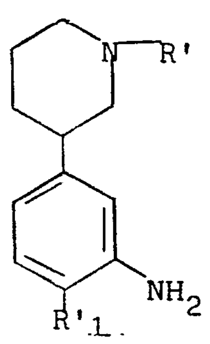

(II')

dans laquelle R' et $R'_1$ ont la signification de R et $R_1$ respectivement, à l'exception de l'hydrogène pour R et de hydroxy pour $R_1$, et fait réagir l'halogénamine avec le méthylthioacétate d'éthyle en présence d'une base, puis avec un acide, pour obtenir un produit de formule (III') :

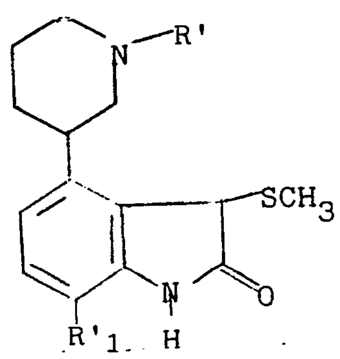

(III')

dans laquelle R' et $R'_1$ ont la signification déjà indiquée, que l'on désulfure, pour obtenir un produit de formule ($I_D$) tel que défini ci-dessus, que soit l'on isole et, si désiré, salifie, soit l'on soumet à l'action d'un agent de déblocage de l'hydroxyle, pour obtenir un produit de formule ($I_C$) tel que défini ci-dessus, que ou bien l'on isole et, si désiré, salifie, ou bien dans le cas où R' représente un radical benzyle, l'on soumet à l'action d'un agent de déblocage du benzyle, pour obtenir un produit de formule ($I_B$) tel que défini ci-dessus, soit dans le cas où R' représente un radical benzyle, l'on soumet ledit produit de formule ($I_D$), à l'action d'un agent de déblocage du benzyle, pour obtenir un produit de formule ($I_A$) tel que défini ci-dessus, que ou bien l'on isole et, si désiré, salifie, ou bien l'on soumet à l'action d'un agent de déblocage de l'hydroxyle, pour obtenir un produit de formule ($I_B$) tel que défini ci-dessus, que l'on isole et, si désiré, salifie.

**8.** Médicaments, caractérisés en ce qu'ils sont constitués par les nouveaux dérivés du 7-hydroxy indole tels que définis par la formule I de la revendication 1 ainsi que par leurs sels d'addition avec les acides pharmaceutiquement acceptables.

**9.** Médicaments, caractérisés en ce qu'ils sont constitués par les nouveaux dérivés du 7-hydroxy indole tels que définis dans l'une des revendications 2 ou 3 ainsi que par leurs sels d'addition avec les acides pharmaceutiquement acceptables.

**10.** Médicaments, caractérisés en ce qu'ils sont constitués par le dérivé du 7-hydroxy indole tel que défini à la revendication 4 ainsi que par ses sels d'addition avec les acides pharmaceutiquement acceptables.

**11.** Compositions pharmaceutiques, caractérisées en ce qu'elles renferment à titre de principe actif, l'un au moins des médicaments, tels que définis à l'une des revendications 8, 9 ou 10.

**12.** Les dérivés de formule II ou II' :

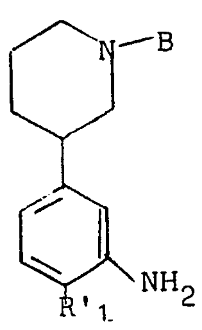

(II)

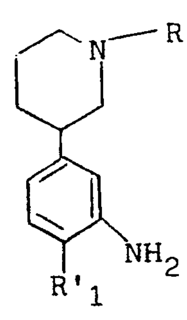

(II')

dans lesquelles B, R' et $R'_1$ ont la signification déjà indiquée ci-dessus et leurs halogénamines.

## Claims

**1.** The derivatives of 7-hydroxy indole as well as their addition salts with mineral or organic acids,

characterized in that they correspond to general formula (I):

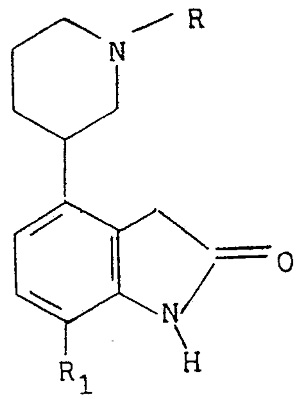

(I)

in which R represents a hydrogen atom, an alkyl radical containing 1 to 5 carbon atoms, a cycloalkylalkyl radical containing 4 to 7 carbon atoms, an aralkyl radical containing 7 to 12 carbon atoms, optionally substituted by one or more radicals chosen from the group constituted by alkyl radicals containing 1 to 5 carbon atoms, alkoxy radicals containing 1 to 5 carbon atoms, alkenyl or alkynyl radicals containing 3 to 5 carbon atoms and halogen atoms, it being understood that the multiple bond cannot be found between the carbons in position alpha and beta of the nitrogen atom and $R_1$ represents a hydroxy radical, an alkoxy radical containing 1 to 5 carbon atoms, a phenylalkyloxy radical containing 7 to 9 carbon atoms or a phenoxy radical.

**2.** The derivatives of 7-hydroxy indole as defined by formula (I) of claim 1 as well as their addition salts with mineral or organic acids, characterized in that $R_1$ represents a hydroxy radical.

**3.** The derivatives of 7-hydroxy indole according to claim 2 as well as their addition salts with mineral or organic acids, characterized in that R represents a hydrogen atom or an alkyl radical containing 1 to 5 carbon atoms.

**4.** The derivative of formula (I) according to claim 1 of which the name follows:
- 1,3-dihydro-7-hydroxy-4-(3-piperidinyl)-2H-indol-2-one as well as its addition salts with mineral or organic acids.

**5.** Preparation process for derivatives of 7-hydroxy indole as defined by formula (I) of claim 1 as well as their salts, characterized in that a derivative of formula (II):

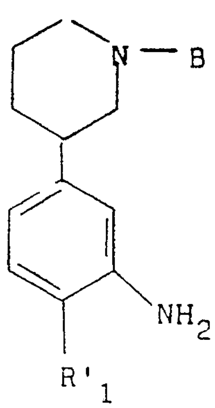

(II)

in which B represents a reversible blocking agent of a secondary amine and $R'_1$ has the value of $R_1$ with the exception of the hydroxy, is activated in the form of the halogenamine and the halogenamine is reacted with ethyl methylthioacetate in the presence of a base, then with an acid in order to obtain a product of formula (III):

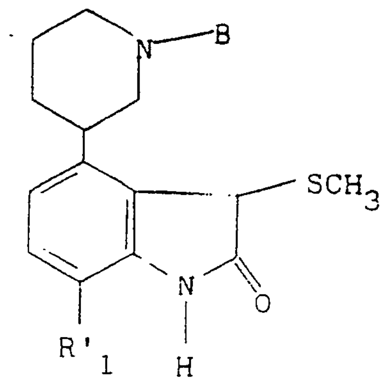

(III)

in which B and $R'_1$ have the meaning already indicated, which is desulphurized, in order to obtain a product of formula (IV):

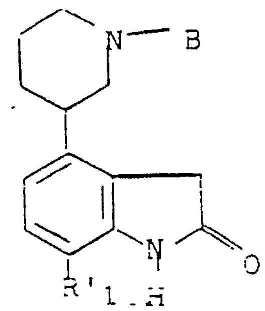

(IV)

in which B and $R'_1$ have the meaning already indicated, which is then reacted with a deblocking agent of the secondary amine, in order to obtain a product of formula $I_A$:

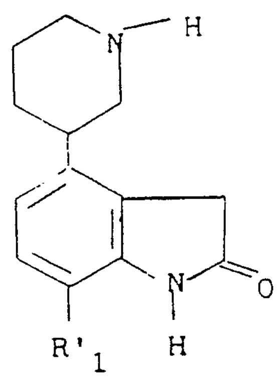

($I_A$)

in which $R'_1$ has the meaning already indicated, which is either isolated and salified, if desired, or is subjected to the action of a blocking agent of the hydroxyl, in order to obtain a product of formula ($I_B$):

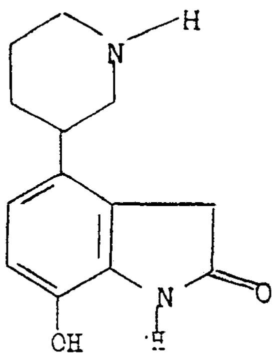

(I$_B$)

which is either isolated and salified, if desired, or is reacted an agent capable of grafting an R' radical, R' having the meaning of R with the exception of hydrogen, in order to obtain a product of formula $I_C$:

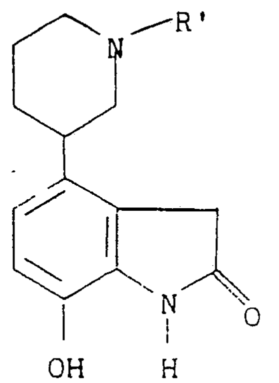

(I$_C$)

in which R' has the meaning already indicated, which is isolated and, if desired, salified, or said product of formula ($I_A$) is reacted with an agent capable of grafting an R' radical as defined above, in order to obtain a product of formula ($I_D$):

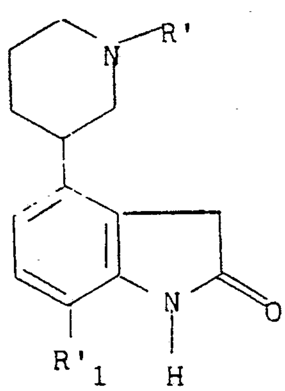

(I$_D$)

in which R' and $R'_1$ have the meaning already indicated, which is either isolated and, if desired, salified or is subjected to the action of a deblocking agent of the hydroxyl in order to obtain a product of formula ($I_C$) as defined above, which is isolated and, if desired salified.

6. Process according to claim 5, characterized in that:
   - the blocking agent of the secondary amine is a trifluoroacetyl radical;
   - the halogenamine is a chloramine.

7. Variant of the preparation process for the derivatives of 7-hydroxy indole as defined by formula I of claim 1 as well as their salts, characterized in that a derivative of formula (II'):

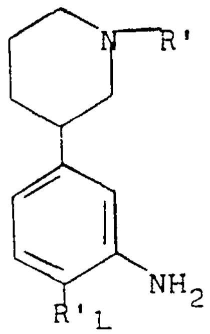

(II')

in which R' and $R'_1$ have the meaning of R and $R_1$ respectively, with the exception of the hydrogen for R and the hydroxy for $R_1$, is activated in the form of the halogenamine, and the halogenamine is reacted with ethyl methylthioacetate in the presence of a base, then with an acid, in order to obtain a product of formula (III'):

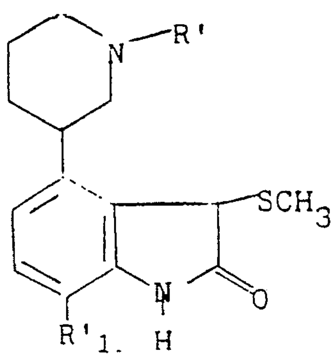

(III')

in which R' and $R'_1$ have the meaning already indicated, which is desulphurized, in order to obtain a product of formula ($I_D$) as defined above, which is either isolated and, if desired, salified, or is subjected to the action of a deblocking agent of the hydroxyl, in order to obtain a product of formula ($I_C$) as defined above, which is either isolated and, if desired, salified, or in the case where R' represents a benzyl radical is subjected to the action of a deblocking agent of the benzyl, in order to obtain a product of formula ($I_B$) as defined above,
or in the case where R' represents a benzyl radical, said product of formula ($I_D$) is subjected to the action of a deblocking agent of the benzyl, in order to obtain a product of formula ($I_A$) as defined above, which is either isolated and, if desired, salified, or is subjected to the action of a deblocking agent of the hydroxyl, in order to obtain a product of formula ($I_B$) as defined above, which is isolated and, if desired, salified.

**8.** Medicaments, characterized in that they are constituted by the new derivatives of 7-hydroxy indole as defined by formula I of claim 1 as well as their addition salts with pharmaceutically acceptable acids.

**9.** Medicaments, characterized in that they are constituted by the new derivatives of 7-hydroxy indole as defined in one of claims 2 or 3 as well as by their addition salts with pharmaceutically acceptable acids.

**10.** Medicaments, characterized in that they are constituted by the derivative of 7-hydroxy indole as defined in claim 4 as well as by their addition salts with pharmaceutically acceptable acids.

**11.** Pharmaceutical compositions, characterized in that they contain as active ingredient, at least one of the medicaments as defined in one of claims 8, 9 or 10.

**12.** The derivatives of formula (II) or (II'):

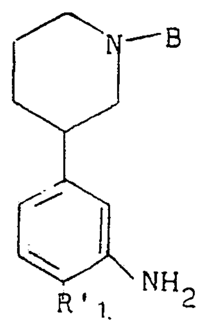

(II)

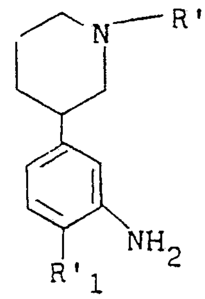

(II')

in which B, R' and $R'_1$ have the meaning already indicated above and their halogenamines.

## Ansprüche

1. 7-Hydroxyindolderivate sowie deren Additionssalze mit Mineral- oder organischen Säuren, dadurch gekennzeichnet, daß sie der allgemeinen Formel (I):

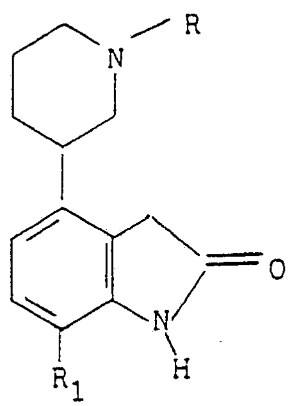

(I)

entsprechen, worin R für ein Wasserstoffatom, einen Alkylrest mit 1 bis 5 Kohlenstoffatomen, einen Cycloalkylalkylrest mit 4 bis 7 Kohlenstoffatomen, einen Aralkylrest mit 7 bis 12 Kohlenstoffatomen, der gegebenenfalls durch einen oder mehrere Reste, ausgewählt unter den Alkylresten mit 1 bis 5 Kohlenstoffatomen, den Alkoxyresten mit 1 bis 5 Kohlenstoffatomen, den Alkenyl- oder Alkinylresten mit 3 bis 5 Kohlenstoffatomen und den Halogenatomen, substituiert ist, steht mit der Maßgabe, daß die Mehrfachbindung sich nicht zwischen den Kohlenstoffatomen in $\alpha$- und $\beta$-Stellung zu dem Stickstoffatom befinden kann, und $R_1$ einen Hydroxyrest, einen Alkoxyrest mit 1 bis 5 Kohlenstoffatomen, einen Phenylalkyloxyrest mit 7 bis 9 Kohlenstoffatomen oder Phenoxy bedeutet.

2. 7-Hydroxyindolderivate der Formel (I) gemäß Anspruch 1 sowie deren Additionssalze mit Mineral- oder organischen Säuren, dadurch gekennzeichnet, daß $R_1$ für einen Hydroxyrest steht.

3. 7-Hydroxyindolderivate gemäß Anspruch 2 sowie deren Additionssalze mit Mineral- oder organischen Säuren, dadurch gekennzeichnet, daß R für ein Wasserstoffatom oder einen Alkylrest mit 1 bis 5 Kohlenstoffatomen steht.

4. Derivat der Formel (I) gemäß Anspruch 1 mit der folgenden Bezeichnung: 1,3-Dihydro-7-hydroxy-4-(3-piperidinyl)-(2H-indol-2-on) sowie dessen Additionssalze mit Mineral- oder organischen Säuren.

5. Verfahren zur Herstellung der 7-Hydroxyindolderivate der Formel (I) gemäß Anspruch 1 sowie von deren Salzen, dadurch gekennzeichnet, daß man ein Derivat der Formel (II):

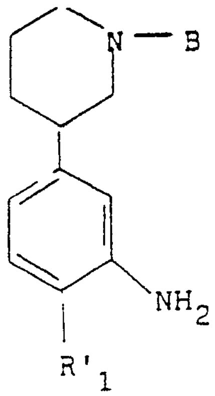

(II)

worin B für ein Mittel zur reversiblen Blockierung eines sekundären Amins steht, und $R'_1$ die Bedeutung von $R_1$ mit Ausnahme von Hydroxy besitzt, in Form eines Halogenamins aktiviert und das Halogenamin mit Methylthioessigsäureethylester in Anwesenheit einer Base, danach mit einer Säure umsetzt, um zu einem Produkt der Formel (III):

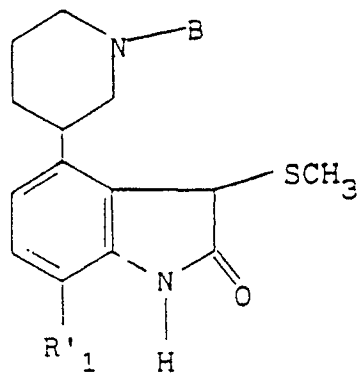

(III)

zu gelangen, worin B und $R'_1$ die angegebene Bedeutung besitzen, welches man entschwefelt, um zu einem Produkt der Formel (IV):

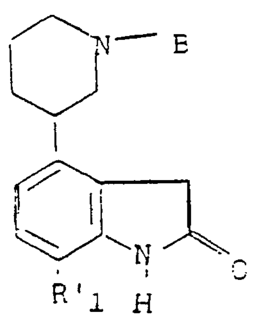

(IV)

zu gelangen, worin B und $R'_1$ die angegebene Bedeutung besitzen, hiernach mit einem Mittel zur Deblockierung des sekundären Amins umsetzt, um zu einem Produkt der Formel ($I_A$):

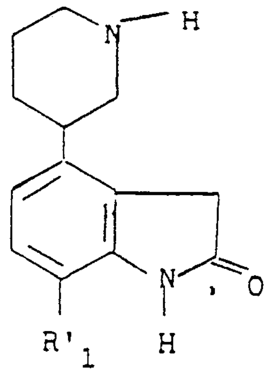

($I_A$)

zu gelangen, worin $R'_1$ die angegebene Bedeutung besitzt, welches man entweder isoliert und gewünschtenfalls in ein Salz überführt oder der Einwirkung eines Deblockierungsmittels für die Hydroxylgruppe unterzieht, um zu einem Produkt der Formel ($I_B$):

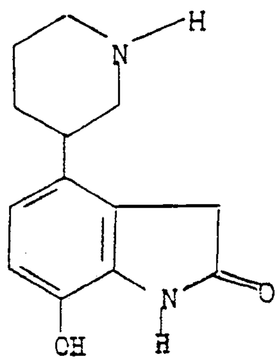

($I_B$)

zu gelangen, welches man isoliert und gewünschtenfalls in ein Salz überführt oder mit einem Mittel umsetzt, das befähigt ist, einen Rest R' einzuführen, wobei R' die Bedeutung von R mit Ausnahme von Wasserstoff besitzt, um zu einem Produkt der Formel ($I_C$):

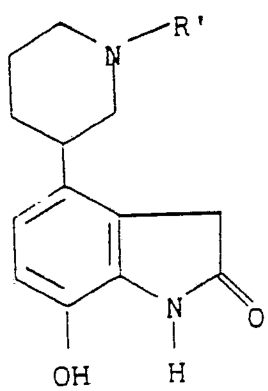

($I_C$)

zu gelangen, worin R' die angegebene Bedeutung besitzt, welches man isoliert und gewünschtenfalls in ein Salz überführt oder das Produkt der Formel ($I_A$) mit einem Mittel umsetzt, das befähigt ist, einen Rest R', wie vorstehend definiert, einzuführen, um zu einem Produkt der Formel ($I_D$):

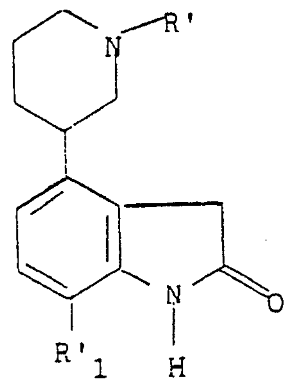

$(I_D)$

zu gelangen, worin R' und $R'_1$ die angegebene Bedeutung besitzen, welches man entweder isoliert und gewünschtenfalls in ein Salz überführt oder der Einwirkung eines Deblockierungsmittels für die Hydroxylgruppe unterzieht, um zu einem Produkt der Formel ($I_C$), wie vorstehend definiert, zu gelangen, welches man isoliert und gewünschtenfalls in ein Salz überführt.

**6.** Verfahren gemäß Anspruch 5, dadurch gekennzeichnet, daß
- das Blockierungsmittel für das sekundäre Amin ein Trifluoracetylrest ist;
- das Halogenamin ein Chloramin ist.

**7.** Abänderung des Verfahrens zur Herstellung der 7-Hydroxyindolderivate der Formel (I) gemäß Anspruch 1 sowie von deren Salzen, dadurch gekennzeichnet, daß man ein Derivat der Formel (II'):

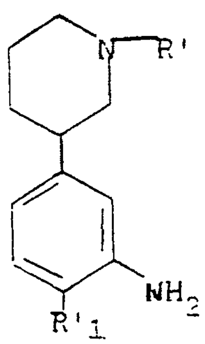

(II')

worin R' und $R'_1$ die Bedeutung von R bzw. $R_1$ mit Ausnahme von Wasserstoff für R und Hydroxy für $R_1$ besitzen, in Form des Halogenamins aktiviert und das Halogenamin mit dem Methylthioessigsäureethylester in Gegenwart einer Base, danach mit einer Säure umsetzt, um zu einem Produkt der Formel (III'):

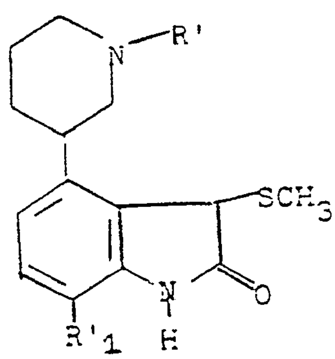

(III')

zu gelangen, worin $R^1$ und $R'_1$ die angegebene Bedeutung besitzen, welches man entschwefelt, um zu einem Produkt der Formel ($I_D$), wie vorstehend definiert, zu gelangen, welches man entweder isoliert

und gewünschtenfalls in ein Salz überführt oder der Einwirkung eines Deblockierungsmittels für die Hydroxylgruppe unterzieht, um zu einem Produkt der Formel ($I_C$), wie vorstehend definiert, zu gelangen, welches man entweder isoliert und gewünschtenfalls in ein Salz überführt oder, wenn R' einen Benzylrest bedeutet, der Einwirkung eines Deblockierungsmittels für die Benzylgruppe unterzieht, um zu einem Produkt der Formel ($I_B$), wie vorstehend definiert, zu gelangen oder, wenn R' für einen Benzylrest steht, man das Produkt der Formel ($I_D$) der Einwirkung eines Deblockierungsmittels für die Benzylgruppe unterzieht, um zu einem Produkt der Formel ($I_A$), wie vorstehend definiert, zu gelangen, welches man entweder isoliert und gewünschtenfalls in ein Salz überführt oder der Einwirkung eines Deblockierungsmittels für die Hydroxylgruppe unterzieht, um zu einem Produkt der Formel ($I_B$), wie vorstehend definiert, zu gelangen, welches man isoliert und gewünschtenfalls in ein Salz überführt.

**8.** Arzneimittel, dadurch gekennzeichnet, daß sie aus den neuen 7-Hydroxyindolderivaten der Formel (I) gemäß Anspruch 1 sowie aus deren Additionssalzen mit pharmazeutisch verträglichen Säuren bestehen.

**9.** Arzneimittel, dadurch gekennzeichnet, daß sie aus den neuen 7-Hydroxyindolderivaten gemäß einem der Ansprüche 2 oder 3 sowie aus deren Additionssalzen mit pharmazeutisch verträglichen Säuren bestehen.

**10.** Arzneimittel, dadurch gekennzeichnet, daß sie aus dem 7-Hydroxyindolderivat gemäß Anspruch 4 sowie aus dessen Additionssalzen mit pharmazeutisch verträglichen Säuren bestehen.

**11.** Pharmazeutische Zusammensetzungen, dadurch gekennzeichnet, daß sie als Wirkstoff zumindest eines der Arzneimittel gemäß einem der Ansprüche 8, 9 oder 10 enthalten.

**12.** Derivate der Formel (II) oder (II'):

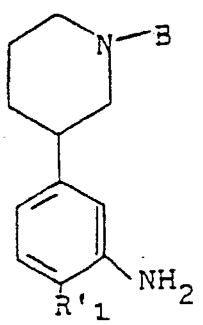
(II)

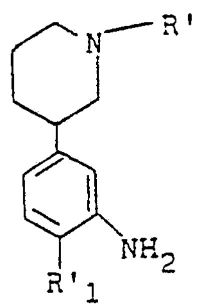
(II')

worin B, R' und $R'_1$ die angegebene Bedeutung besitzen, und deren Halogenamine.